# EUROPÄISCHE PATENTSCHRIFT

(11) **EP 2 109 476 B1**
(45) Veröffentlichungstag und Bekanntmachung des Hinweises auf die Patenterteilung: **02.07.2014**
(21) Anmeldenummer: 07855999.4
(22) Anmeldetag: 24.10.2007
(51) Int. Cl.: A61M 16/08, A61M 16/10

(54) **ATEMSCHLAUCHSYSTEM MIT EINEM HEIZELEMENT**
BREATHING TUBE SYSTEM COMPRISING A HEATING ELEMENT
SYSTÈME DE TUYAU RESPIRATOIRE MUNI D'UN ÉLÉMENT CHAUFFANT

(30) Priorität: 10.11.2006 DE 102006052997
(43) Veröffentlichungstag der Anmeldung: 21.10.2009
(73) Patentinhaber: Dräger Medical GmbH, 23558 Lübeck (DE)
(72) Erfinder: KRATZENSTEIN, Markus, 23570 Lübeck (DE)
(74) Vertreter: UEXKÜLL & STOLBERG
(86) Internationale Anmeldenummer: PCT/DE2007/001920
(87) Internationale Veröffentlichungsnummer: WO 2008/055467

(56) Entgegenhaltungen:
- WO-A-02/32486
- WO-A-2004/105848
- US-A- 5 701 887
- US-B1- 6 167 883

## Beschreibung

Die Erfindung betrifft ein Atemschlauchsystem mit einem Heizelement.

In der klinischen Praxis gibt es verschiedene Atemschlauchsysteme für die Versorgung eines maschinell beatmeten Patienten, in denen Atemgas über die Länge des Schlauches aktiv beheizt wird, um eine zu starke Abkühlung des Atemgases an der oder den Schlauchwandungen zu vermeiden. Dies wird üblicherweise dadurch erreicht, dass ein oder mehrere Heizelemente in den Schlauch eingeführt sind, welche durch Aufprägung eines elektrischen Stromes in einen oder mehrere Widerstandsdrähte Wärmeleistung erzeugen. Die erzeugte Wärme wird an das durchströmende Atemgas insbesondere durch Konvektion abgegeben.

Bei den bekannten Systemen mit Heizdrähten eines definierten Widerstandes sind diese entlang des Schlauches in die Schlauchwände eingearbeitet. Üblicherweise wird ein Heizdraht vom atemgeräteseitigen zum patientenseitigen Anschluss und zurück ausgeführt. Der Heizdraht wird atemgeräteseitig aus dem Atemschlauchsystem herausgeführt und elektrisch kontaktiert. Patientenseitig wird der Heizdraht üblicherweise durch ein Spannelement gehalten, damit er auch während des Betriebes gleichmäßig im Schlauch verteilt bleibt. Das genannte Spannelement schränkt jedoch die Bewegungsfreiheit des Schlauches in längsaxialer Richtung ein.

Dadurch, dass der Heizdraht nur einmal hin- und zurück ausgeführt ist, ist die für die Wärmeabgabe relevante Drahtoberfläche im Betrieb mit einer hohen Temperatur versehen, um eine definierte Wärmeleistung an das durchströmende Atemgas abzugeben. Da die Temperaturen oft zu hoch für das verwendete Schlauchmaterial sind, muss der Widerstand des Heizdrahtes so gewählt werden, dass die Temperaturen limitiert sind, allerdings mit der dann unerwünschten Folge, dass die geforderte Wärmeleistung im Schlauch nicht mehr abgegeben wird.

Bei anderen Atemschlauchsystemen wird dieses Problem dadurch gelöst, dass der Heizdraht im Schlauch als Helix oder als Doppelhelix ausgeführt ist. Eine Doppelhelix bildet sich durch die Eigenspannung des Heizdrahtes oder des Isolationsmaterials aus. Bei diesen Atemschlauchsystemen wird eine relativ große Annäherung an die Schlauchwandung erreicht und ein Mehrfaches der Schlauchlänge kann in den Schlauch eingebracht werden. Jedoch treten durch den beidseitig helixförmigen Verlauf bei diesen Atemschlauchsystemen nach jedem halben Helixumlauf Kreuzungspunkte auf, bei denen zwei Heizdrähte sich kreuzen und aufeinander liegen und sich somit berühren. An diesen Stellen können sich starke Temperaturüberhöhungen ausbilden durch die fehlende Wärmeabführung an den Berührungspunkten. Dies stellt insbesondere bei Schlauchmaterialien mit einem niedrigen Schmelzpunkt ein Risiko des Schmelzens dar und birgt auch ein erhöhtes Entflammbarkeitsrisiko für das verwendete Schlauchmaterial.

Ein weiterer Nachteil der Doppelhelix ist das Auftreten von stark turbulenten Strömungen im Atemgas verbunden mit einem hohen Strömungswiderstand im Schlauch. Die Verwirbelung im Atemgas entsteht durch den senkrecht zur Atemgasströmung verlaufenden Draht.

Die derzeit im Markt erhältlichen Atemschlauchsysteme haben den weiteren Nachteil, dass der oder die Heizdrähte in der Helix- oder Doppelhelix-Ausbildung mit einem Werkzeug in den Schlauch eingezogen werden müssen, so dass leicht Beschädigungen des Schlauches auftreten können.

Ein als Atemgasanfeuchter ausgebildetes Atemschlauchsystem geht aus der US 3,871,373 hervor, wobei Wasser in einem Wasserdampf abgebenden Schlauch in dem eigentlichen Atemschlauchsystem in Längsrichtung hin und zurück geführt ist.

Ein Atemschlauchsystem mit einem zentral im Abstand von der Schlauchwandung geführten Heizelement wird in der DE 196 47 548 C2 beschrieben, wobei die Heizleitung mittels mindestens eines thermisch isolierenden Abstandshalters gehalten ist.

Die WO 2004/105848 A1 offenbart eine Heizeinrichtung für einen Atemschlauch. Bei einem ersten Ausführungsbeispiel verläuft ein Heizdraht im Inneren eines Atemschlauchs in Form einer Schlaufe vom geräteseitigen Ende zum patientenseitigen Ende und wieder zurück. Hierbei werden die beiden Drähte durch Abstands-Clips beabstandet gehalten und gegen die Innenwand des Schlauchs gedrückt. Folglich ist nur ein Heizdraht vorgesehen, der nur einmal hin- und zurück verläuft. Bei einem weiteren Ausführungsbeispiel sind die Abstands-Clips durch ein durchgehendes Band ersetzt. Hierbei sind eben-falls nur zwei Heizdrähte vorgesehen. Bei einer weiteren Ausführung ist ein extrudierter durchgehender Haltekörper mit einem kreuzförmigen Querschnitt vorgesehen. Hier sind statt der zwei Heizdrähte nun vier Heizdrähte vorgesehen. Ein entscheidender Nachteil dieser Lösung besteht allerdings darin, dass die Biegeeigenschaften des Atemschlauchs durch den durchgehenden kreuzförmigen Einsatz stark beeinträchtig werden. Schließlich werden insgesamt vier Atemluftkanäle gebildet, was sich nachteilig auf die Atemluftströmungscharakteristik auswirkt.

Die US 5,701,887 zeigt ebenfalls einem Atemschlauch, in den ein Haltekörper mit zwei Nuten eingesetzt ist, um einen hin- und zurück verlaufenden Heizdraht zu halten.

Die US 6,167,883 B1 beschreibt einen Atemschlauch mit einer Heizeinrichtung in der Form eines durchgehenden Streifens in den ein hin- und zurück verlaufender Heizdraht eingebettet ist.

Die Aufgabe der Erfindung besteht in der Bereitstellung eines verbesserten, einfach auszuführenden Atemschlauchsystems mit einem Heizelement, so dass eine möglichst gleichförmige Wärmeabgabe an das durchströmende Atemgas bei relativ niedriger Temperatur des Heizelementes möglich ist.

Die Lösung der Aufgabe erhält man mit den Merkmalen von Anspruch 1. Die Unteransprüche geben bevorzugte Ausbildungen des Atemschlauchsystems von Anspruch 1 an.

Das Atemschlauchsystem nach Anspruch 1 hat den wesentlichen Vorteil, dass eine Heizdrahtlänge entsprechend einem Mehrfachen der Atemschlauchlänge im Schlauch integriert ist und dadurch mit einer niedrigeren Heizdrahttemperatur gearbeitet werden kann, so dass keine Überhitzungseffekte im Schlauchmaterial auftreten können und eine gute Wärmeübertragung an die durchströmenden Atemgase erreicht wird. Dies wird im Ergebnis dadurch erreicht, dass die Heizdrähte im Schlauch im Wesentlichen parallel zur Längsrichtung des Schlauches verlaufen und mehrere Hin- und Rückführungen erfolgen und berührende Überkreuzungen weitgehend ausgeschlossen sind. Hierzu werden die Heizdrähte mit Hilfe mehrerer Spreizelemente entlang der Schlauchlänge beabstandet geführt, die insbesondere mit den Heizdrähten in den Schlauch eingebracht sind. Die Spreizelemente sind so ausgeführt, dass sie den vorzugsweise einen Heizdraht seitlich möglichst eng an der Schlauchwandung führen, so dass sich dort durch die abgegebene Wärme kein Kondensat bildet. Durch den gewählten Abstand der Spreizelemente in Längsrichtung des Schlauches und durch die Eigensteifigkeit des Heizdrahtes wird verhindert, dass der Heizdraht im Schlauch zusammensackt und es demzufolge zu einer Knäuelbildung kommt.

Im Folgenden wird ein Ausführungsbeispiel der Erfindung mit Hilfe der Figuren erläutert.

Es zeigen:
- Figur 1: einen Schnitt durch einen Schlauch eines Atemschlauchsystems mit einem Spreizelement mit vier Spreizarmen für die Aufnahme des Heizdrahtes und
- Figur 2: eine Ansicht eines Atemschlauchsystems im Längsschnitt.

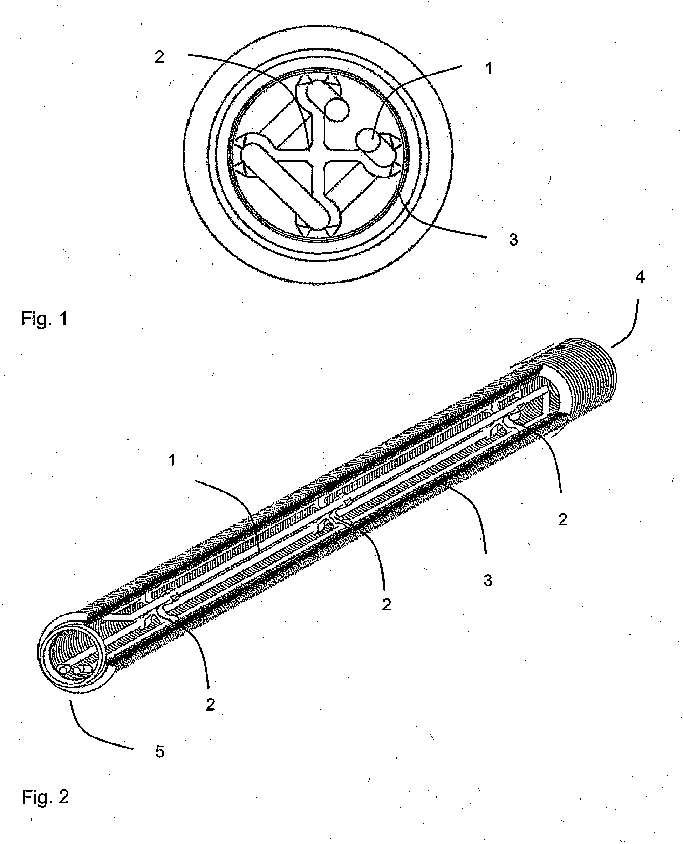

Die Spreizelemente 2, welche in der Schlauchwandung 3 gemäß Figur 1 abgestützt sind, weisen mehrere, beispielsweise vier, möglichst schmale Spreizarme auf für die Aufnahme und Führung eines Heizdrahtes 1 möglichst an der Schlauchwandung 3 und passen sich in die Innenkontur des Schlauches ein. Die Anzahl der Führungselemente hängt von der Anzahl der Verläufe des Heizdrahtes 1 in Längsrichtung des Schlauches ab. In der dargestellten Ausführung gemäß den Figuren 1 und 2 läuft der Heizdraht 1 zweimal vom Anästhesie- oder Beatmungsgerät bzw. vom geräteseitigen Ende 5 zum patientenseitigen Ende 4 und zurück. Der elektrisch leitende Teil des Heizdrahtes 1 besteht speziell aus mehreren Litzen, um das Auftreten von Widerstandserhöhungen bei Kabelbruch zu vermeiden. Die Litzen sind vorzugsweise mit einer gemeinsamen äußeren Isolierschicht aus temperaturbeständigem Silikon oder einem anderen geeigneten Material wie Polypropylen überzogen.
Der Heizdraht 1 ist entlang der Schlauchlängsrichtung durch beispielsweise drei beabstandete Spreizelemente 2 gehalten, wobei sich eins am bzw. vor dem geräteseitigen Ende 5 befindet, eins am bzw. vor dem patientenseitigen Ende 4 und eins etwa in der Mitte des Schlauchs an der Schlauchwandung 3. Der Heizdraht 1 ist beispielsweise vierfach in den Schlauch eingelegt und in die Spreizelemente 2 eingespannt, so dass sich aus Gerätesicht eine doppelte Hin- und Rückführung des Heizdrahtes 1 ergibt. Beide elektrischen Kontakte werden am geräteseitigen Ende 5 aus dem Schlauch geführt und im nicht weiter dargestellten Anästhesie- oder Beatmungsgerät kontaktiert. Die Länge des Atemschlauchsystems beträgt etwa 1 bis 1,5 m, der Durchmesser je nach Anwendungsfall etwa 10 bis 30 mm.

Als wesentliche Vorteile des vorliegenden Atemschlauchsystems sind zu nennen:

Durch das vier- oder mehrfache Einlegen des Heizdrahtes 1 über die Schlauchlänge und die im Wesentlichen parallele Führung an der inneren Schlauchwandung 3 in Längsrichtung des Schlauches wird eine verbesserte Verteilung der Wärmeabgabe an das durchströmende Atemgas erreicht. Durch die parallele Führung der Heizdrähte 1 im Schlauch wird außerdem erreicht, dass der Strömungswiderstand im Schlauchsystem akzeptabel niedrig bleibt, und durch die parallele Positionierung der Spreizelemente 2 im Schlauch wird eine nahezu laminare Strömung erzielt. Gleichzeitig werden die Heizdrähte 1 weitgehend berührungslos geführt. Zudem kann der Heizdraht 1 mit den führenden Spreizelementen 2 problemlos manuell in den Schlauch eingeführt werden.

## Patentansprüche

1. Atemschlauchsystem mit einem Heizelement,
wobei ein mehrfach in Längsrichtung eines Schlauches hin- und zurück verlaufender Heizdraht (1) mit Drahtanschlüssen an einem Ende in dem Schlauch mittels mehrerer, voneinander in Längsrichtung des Schlauches beabstandeter Spreizelemente (2) parallel gehalten wird,
der Heizdraht (1) mindestens viermal in Längsrichtung des Schlauches verläuft,
die Spreizelemente (2) Führungselemente für die Aufnahme des Heizdrahtes (1) an der inneren Schlauchwandung (3) des Schlauches aufweisen und die Spreizelemente (2) mit dem Heizdraht (1) in Längsrichtung des Schlauches verschiebbar sind.

2. Atemschlauchsystem nach Anspruch 1, **dadurch gekennzeichnet, dass** mindestens drei Spreizelemente (2) beabstandet über die Schlauchlänge angeordnet sind und dass jedes Spreizelement (2) mindestens vier Führungselemente für die Aufnahme des Heizdrahtes (1) aufweist.

3. Atemschlauchsystem nach einem der vorhergehenden Ansprüche, **dadurch gekennzeichnet, dass** die Drahtanschlüsse am geräteseitigen Ende (5) des Schlauches angeordnet sind.

4. Atemschlauchsystem nach einem der vorhergehenden Ansprüche, **dadurch gekennzeichnet, dass** der Heizdraht (1) aus mehreren Litzen besteht, die vorzugsweise mittels eines elektrisch isolierenden Kunststoffes wie insbesondere Silikon gemeinsam überzogen sind.

5. Atemschlauchsystem nach einem der vorhergehenden Ansprüche, **dadurch gekennzeichnet, dass** die Spreizelemente (2) mit mehreren Spreizarmen mit je einem Führungselement entsprechend der Anzahl der aufzunehmenden Heizdrähte (1) ausgestattet sind.

6. Atemschlauchsystem nach Anspruch 5, **dadurch gekennzeichnet, dass** die Spreizelemente (2) äquidistant angeordnet sind, insbesondere in Form eines Kreuzes ausgebildet sind.

7. Atemschlauchsystem nach einem der vorhergehenden Ansprüche, **dadurch gekennzeichnet, dass** die Länge 1 bis 1,5 Meter und der Innendurchmesser etwa 10 bis 30 Millimeter betragen.

## Claims

1. Breathing tube system comprising a heating element,
wherein a heating wire (1) with wire terminals at one end extending several times to and fro in the longitudinal direction of said tube, and being held in parallel in the tube by a plurality of spreading elements (2) spaced apart from each other in the longitudinal direction of the tube,
the heating wire (1) extending at least four times in the longitudinal direction of the tube,
the spreading elements (2) having guide elements for receiving the heating wire (1) at the inner tube wall (3) of the tube, and the spreading elements (2) being displaceable with the heating wire (1) in the longitudinal direction of the tube.

2. Breathing tube system in accordance with claim 1, **characterized in that** at least three spreading elements (2) are arranged at spaced locations from one another over the length of the tube, and that each spreading element (2) has at least four guide elements for receiving the heating wire (1).

3. Breathing tube system in accordance with any of the preceding claims, **characterized in that** the wire terminals are arranged at the device-side end of the tube.

4. Breathing tube system in accordance with any of the preceding claims, **characterized in that** the heating wire (1) comprises a plurality of strands which are coated together by an electrically insulating plastic, in particular a silicone material.

5. Breathing tube system in accordance with any of the preceding claims, **characterized in that** the spreading elements (2) are provided with a plurality of spreading arms each having a guide element corresponding to the number of heating wires (1) to be received.

6. Breathing tube system in accordance with claim 5, **characterized in that** the spreading elements (2) are arranged equidistantly, in particular in the form of a cross.

7. Breathing tube system in accordance with any of the preceding claims, **characterized in that** the length is from 1 m to 1,5 m, and the internal diameter is from 10 mm to 30 mm.

## Revendications

1. Système de tuyau respiratoire comprenant un élément chauffant,
dans lequel un filament chauffant (1) muni de connexions de filament à une extrémité et faisant plusieurs allers-retours dans le sens longitudinal d'un tuyau est maintenu parallèle dans le tuyau au moyen de plusieurs éléments écarteurs (2) placés à distance les uns des autres dans le sens longitudinal du tuyau,
le filament chauffant (1) s'étend au moins quatre fois dans le sens longitudinal du tuyau,
les éléments écarteurs (2) présentent des éléments de guidage pour la réception du filament chauffant (1) sur la paroi interne (3) du tuyau, et les éléments écarteurs (2) peuvent être déplacés avec le filament chauffant (1) dans le sens longitudinal du tuyau.

2. Système de tuyau respiratoire selon la revendication 1, **caractérisé en ce qu'**au moins trois éléments écarteurs (2) sont disposés à distance sur la longueur du tuyau et **en ce que** chaque élément écarteur (2) présente au moins quatre éléments de guidage pour la réception du filament chauffant (1).

3. Système de tuyau respiratoire selon l'une des revendications précédentes, **caractérisé en ce que** les connexions de filament sont disposées à l'extrémité (5) du tuyau située côté appareil.

4. Système de tuyau respiratoire selon l'une des revendications précédentes, **caractérisé en ce que** le filament chauffant (1) est constitué de plusieurs brins qui sont de préférence revêtus ensemble d'une matière plastique électriquement isolante, par exemple notamment de la silicone.

5. Système de tuyau respiratoire selon l'une des revendications précédentes, **caractérisé en ce que** les éléments écarteurs (2) sont équipés de plusieurs bras écarteurs avec respectivement un élément de guidage en fonction du nombre des filaments chauffants (1) à recevoir.

6. Système de tuyau respiratoire selon la revendication 5, **caractérisé en ce que** les éléments écarteurs (2) sont disposés de façon équidistante, en particulier sous la forme d'une croix.

7. Système de tuyau respiratoire selon l'une des revendications précédentes, **caractérisé en ce que** la longueur est comprise entre 1 et 1,5 mètre et le diamètre intérieur est compris entre environ 10 et 30 millimètres.
